# EUROPÄISCHE PATENTANMELDUNG

(11) **EP 4 477 402 A1**
(43) Veröffentlichungstag der Anmeldung: **18.12.2024**
(21) Anmeldenummer: 24000072.9
(22) Anmeldetag: 11.06.2024
(51) Int. Cl.: B30B 5/06, B07C 5/34, B27N 3/18, B30B 15/26, G01N 23/083, G01N 33/46

(54) **VORRICHTUNG UND VERFAHREN ZUR DETEKTION VON FREMDKÖRPERN IN EINER GESTREUTEN MATTE**

(30) Priorität: 13.06.2023 DE 102023002398
(71) Anmelder: Siempelkamp Maschinen- und Anlagenbau GmbH, 47803 Krefeld (DE)
(72) Erfinder: Krumbach-Voß, Rainer, DE-47918 Tönisvorst (DE); Robles Malagrino, Pedro, DE-47803 Krefeld (DE); Schürmann, Klaus, DE-41363 Jüchen (DE); Staub, Günter, DE-47839 Krefeld (DE)

(57) **Zusammenfassung**

Die Erfindung betrifft eine Vorrichtung (21) und ein Verfahren zur Detektion von Fremdkörpern in einer auf eine Fördereinrichtung (4) gestreuten Matte (5) im Zuge einer Werkstoffplattenherstellung, wobei die Matte (5) zumindest teilweise aus Fasern oder Spänen und Bindemitteln besteht, welche unter Temperaturerhöhung verpresst werden, wobei zumindest eine mit elektromagnetischen oder Ultraschall-Wellen oder mit Magnetfeldern oder mit Radiometrie arbeitende erste Sensoreinrichtung (6) vorgesehen ist, die auf einen oder mehrere räumliche Messbereiche (22) der Matte gerichtet ist, um Fremdkörper (10) zu erkennen. Um besser abschätzen zu können, wann ein Fremdkörper das Stahlband (18a, 18b) einer kontinuierlichen Presse (2) schädigen kann und damit die Schwelle zur Auslösung einer Fremdkörperausschleusung (11) zu erhöhen, ist vorgesehen, dass wenigstens eine zweite Sensoreinrichtung (7) vorgesehen ist, die ebenfalls auf die Messbereiche (22) ausgerichtet ist, und wobei die erste Sensoreinrichtung (6) geeignet ist, eine Materialerkennung des Fremdkörpers (10) vorzunehmen, und die zweite Sensoreinrichtung (7) geeignet ist, die Fremdkörperabmessung zu bestimmen. Ferner betrifft die Erfindung eine mit der Vorrichtung (21) oder dem Verfahren zur Detektion von Fremdkörpern ausgerüstete Produktionsanlage von Werkstoffplatten.

## Beschreibung

Die Erfindung betrifft eine Vorrichtung zur Detektion von Fremdkörpern in einer auf eine Fördereinrichtung gestreuten Matte im Zuge einer Werkstoffplattenherstellung, wobei die Matte zumindest teilweise aus Fasern oder Spänen und Bindemitteln besteht, welche unter Temperaturerhöhung verpresst werden, und wobei zumindest eine mit elektromagnetischen oder Ultraschall-Wellen oder mit Magnetfeldern oder mit Radiometrie arbeitende erste Sensoreinrichtung vorgesehen ist, die auf einen oder mehrere räumliche Messbereiche der Matte gerichtet ist, um Fremdkörper zu erkennen.

Die Erfindung betrifft außerdem ein Verfahren zur Detektion von Fremdkörpern in einer auf eine Fördereinrichtung gestreuten Matte im Zuge einer Werkstoffplattenherstellung, wobei die Matte zumindest teilweise aus Fasern oder Spänen und Bindemitteln besteht, welche unter Temperaturerhöhung verpresst werden, wobei zumindest eine mit elektromagnetischen oder Ultraschall-Wellen oder mit Magnetfeldern oder mit Radiometrie arbeitende erste Sensoreinrichtung verwendet wird, die auf einen oder mehrere räumliche Messbereiche der Matte gerichtet ist, um Fremdkörper zu erkennen.

Die Erfindung betrifft ferner eine Produktionsanlage zur Herstellung von Werkstoffplatten mit einer Streustation zur Streuung einer Matte auf eine Fördervorrichtung, wobei die Matte zumindest teilweise aus Fasern oder Spänen und Bindemitteln besteht, und einer Presse zur Verdichtung der Matte, die die erfindungsgemäße Vorrichtung zur Detektion von Fremdkörpern umfasst, sowie ein Herstellungsverfahren für Werkstoffplatten mit einer Streustation, in der eine Matte auf eine Fördervorrichtung gestreut wird, wobei die Matte zumindest teilweise aus Fasern oder Spänen und Bindemitteln besteht und in einer Presse verdichtet wird, die das erfindungsgemäße Verfahren zur Detektion von Fremdkörpern verwendet.

Unter Werkstoffplatten werden in erster Linie Holzwerkstoffplatten verstanden, die bereits in einer vorzugsweise kontinuierlichen Presse hergestellt werden. Hier kommen beispielsweise Span-, MDF- oder OSB-Platten in Frage, wobei die die Matte beinhaltenden Partikel dann im Wesentlichen Holzfasern oder Holzspäne sind, die mit einem Bindemittel versehen wurden. In neuerer Zeit werden auch andere lignocellulosische Materialien, beispielsweise die Fasern von Einjahrespflanzen (z. B. aus Reisstroh) zum Verpressen von Werkstoffplatten genutzt. Werkstoffplatten können im Sinne der Erfindung aber auch Dämmplatten, Gipsplatten, Kunststoffplatten oder andere Werkstoffplatten sein, deren Partikel mittels umlaufender Bänder in einer kontinuierlichen Presse mit Wärmebeaufschlagung gepresst wurden. Eine solche Presse wird bei der Anmelderin unter dem Namen ContiRoll vermarktet. Dabei werden den Partikeln In der Regel vor der kontinuierlichen Presse Bindemittel beigefügt, damit die Werkstoffplatten eine hohe Stabilität erhalten.

Die Fasern oder Späne werden in einer Streustation auf eine Fördereinrichtung, zumeist auf ein Förderband, auch Formband genannt, gestreut und bildet dort eine Matte, die in Richtung Presse gefördert wird. In der kontinuierlichen Presse geben Hydraulikkomponenten einen hohen Druck über zumeist beheizbare Druckplatten und Rollkörper auf das obere und/oder untere Stahl-Pressenband, zwischen denen die Matte mit gleicher Vorschubgeschwindigkeit wie die der Pressenbänder verdichtet wird. Diese Pressenbänder sind in der Regel nur 0.5 bis 4 mm dick und sehr empfindlich gegen zu hohe punktförmige Drücke. Eine zu große Einbeulung oder Beschädigung kann der Grund dafür sein, dass auf der gesamten Anlage nur noch Ausschuss produziert wird. Ein Wechsel der Pressenbänder ist kosten- und arbeitsintensiv und verständlicherweise unerwünscht. Wenn man berücksichtigt, dass es in der kontinuierlichen Presse zu Verdichtungen und somit auch Dickenänderungen also von der Matte zur Werkstoffplatte, sehr hoch sind, so erkennt man, dass bereits relativ kleine harte Fremdkörper im Inneren der Matte bereits durch die Oberfläche stoßen und die Pressenbänder beschädigen können. Typische Verdichtungswerte in kg/m³ sind bei

| Produkt | Dichte nach Streuung | Dichte nach Presse |
|---|---|---|
| MDF | 18 - 35 | 600 - 900 |
| Span | 140 - 150 | 550 - 750 |
| OSB | 60 - 70 | 500 - 700 |

Insbesondere bei MDF-Platten wird die Dicke der Matte zur Werkstoffplatte demnach so stark verringert, dass selbst kleinere eingeschlossene Fremdkörper leicht durch die Oberfläche stoßen können.

Nun ist es aber so, dass Späne oder Fasern vor der Streuung verschiedenen Prozessen ausgesetzt werden, beispielsweise zerkleinert, gewaschen oder mit Bindemitteln besprüht, bis sie in großen Bunkern vor oder über der Streustation gelagert werden. Vielfach kommt es bei den vielen vorgelagerten Prozessen dazu, dass sich unerwünschte Fremdkörper in den Streuvorgang einbinden und in der Matte verbleiben. Abhängig von der Größe und der Härte des Fremdkörpers, können diese im Verdichtungsprozess der Presse aus der Oberfläche hervortreten und Schäden an den Pressenbändern erzeugen.

Deshalb ist es üblich, die Matte vor dem Einlauf in die Presse zu prüfen. Dazu wird beispielsweise und häufig ein Metalldetektor eingesetzt. Es sind aber auch Anlagen bekannt, wo die Bahn geröntgt wird. Findet man einen Fremdkörper, so wird der Herstellprozess dadurch gestoppt, dass man den betroffenen Teil der Matte mit einer Fremdkörperausschleuseeinrichtung entfernt. Dies kann eine Art Fallschacht sein, der sich öffnet, wenn man das Förderband ein Stück zurückzieht. Aber es sind auch Anlagen bekannt, wo der betreffende kontaminierte Mattenteil vom Förderband abgesaugt oder abgebürstet wird.

In der DE 10 2004 048 275 B3 ist beispielweise die Einsatzmöglichkeit eines Metalldetektors beschrieben. Derartige Metalldetektoren arbeiten induktiv mit einer Senderspule, die von einem Wechselstrom durchflossen wird, der ein magnetisches Wechselfeld bewirkt. Durchsetzt dieses Wechselfeld ein unter der Sonde liegendes leitendes Material, so werden dort Wirbelströme induziert, die ihrerseits ein messbares Magnetfeld erzeugen. Von Metallteilen geht aufgrund ihrer Dichte die höchste Gefahr aus und Metalldetektoren sind relativ kostengünstig. Der Detektor kann einzelne Metallteile genau genug detektieren. Nichteisenmetalle wie Kupfer oder Aluminium fallen jedoch nicht darunter.

Metallteile werden aber auch im Produktionsdurchlauf stark verkleinert (Späne, Staub). Ein Metalldetektor integriert die Metallmasse in seinem Feldbereich und kann nicht zwischen einem einzelnen größeren Körper und sehr vielen kleinen unterscheiden. Das heißt, der Metalldetektor wird auch eine ungefährliche Fläche Metallstaub als Metall-Fremdkörper detektieren und damit die Produktion unnötigerweise unterbrechen.

Auf der anderen Seite kann der Metalldetektor keine ebenfalls schädliche größere Leimverklumpungen oder beispielsweise eine Kunststoffmutter orten.

Andere Anordnungen zur Detektion von Fremdkörpern haben ähnliche Probleme. Eine auf Röntgenstrahlen basierende Sensoreinrichtung mit entsprechender Flächenauflösung kann man auch die Silhouette eines Körpers mit entsprechend höheren Dichten erkennen. Der Scanner kann aber nicht die Höhe und damit das Volumen und die Masse des Körpers erkennen. Somit sind zwar nicht nur metallische Fremdkörper erkennbar, sondern auch solche aus anderen Materialien mit entsprechend hoher Dichte.

Da der Fremdkörperscanner aber nur die Fläche des Fremdkörpers detektieren kann, würde ein entsprechend flacher für das Pressenband unschädlicher Körper eine Fehlauslösung der Fremdkörperausschleuseeinrichtung ergeben.

Neben dem induktiven Metalldetektor und dem Röntgenscanner gibt es viele weitere theoretische Sensoreinrichtungen zur Detektion von Fremdkörpern in einer Matte. Darunter fallen
a) Bildsensoren mit sichtbarem und unsichtbarem Licht
b) Ultraschallsender und -sensoren
c) Sensoren nach dem Magneto-optischen Kerr-Effekt
d) Radioaktive oder -metrische Sensoren
e) Magnetische Sensoren (MRT)

Alle diese möglichen Sensoren und Messverfahren können Bestandteil der Erfindung sein und in der ersten Sensoreinrichtung vorhanden sein oder deren Arbeitsweise verfahrensmäßig darstellen. Aber allen ist auch zu eigen, dass sie den Fremdkörper nicht in Gänze erfassen können und häufig unnötige Produktionsunterbrechungen verursachen würden, um das Pressenband zu schützen.

Es ist deshalb die Aufgabe der Erfindung im Herstellprozess von Werkstoffplatten besser abschätzen zu können, wann ein Fremdkörper das Stahlband einer kontinuierlichen Presse schädigen kann und damit die Schwelle zur Auslösung einer Fremdkörperausschleusung zu erhöhen und demzufolge seltener die Produktion zu unterbrechen.

Die Aufgabe wird vorrichtungsmäßig mit den Merkmalen des Anspruchs 1 und insbesondere dadurch gelöst, dass multimodal wenigstens eine zweite Sensoreinrichtung vorgesehen ist, die ebenfalls auf die Messbereiche ausgerichtet ist, und wobei die erste Sensoreinrichtung geeignet ist, eine Materialerkennung des Fremdkörpers vorzunehmen, und die zweite Sensoreinrichtung geeignet ist, wenigstens eine Fremdkörperabmessung zu bestimmen.

Die Aufgabe wird verfahrensmäßig mit den Merkmalen des Anspruchs 13 und insbesondere dadurch gelöst, dass multimodal wenigstens eine zweite Sensoreinrichtung verwendet wird, die ebenfalls, gegebenenfalls zeitversetzt auf die Messbereiche ausgerichtet ist, wobei mit der ersten Sensoreinrichtung eine Materialerkennung des Fremdkörpers vorgenommen und mit der zweiten Sensoreinrichtung wenigstens eine Fremdkörperabmessung bestimmt wird.

Bei der Kombination aus einer ersten Sensoreinrichtung, die geeignet ist, eine Materialerkennung des Fremdkörpers zumindest näherungsweise vorzunehmen, und einer zweiten Sensoreinrichtung, die geeignet ist, die Fremdkörperabmessung zu bestimmen, kann ein schädlicher Fremdkörper deutlich besser identifiziert werden als bisher.

Dabei kann es Anlagen geben, bei denen in Durchlaufrichtung der Matte zunächst die erste Sensoreinrichtung und erst danach die zweite Sensoreinrichtung zur Detektion verwendet wird. Bei anderen Anlagen kann genau die umgekehrte Reihenfolge sinnvoller sein. Das hängt von dem gestreuten Material und der Dicke bzw. Dichte der Matte ab. In einem Fall wird vorgezogen, zunächst das Material und dann die Abmessungen des Fremdkörpers näher zu bestimmen, in einem anderen Fall kann es sinnvoller sein, zuerst die Abmessungen des Fremdkörpers und dann das Material zu bestimmen. Mit den Begriffen "erste" und "zweite" Sensoreinrichtung ist also lediglich ausgesagt, dass es zwei unterschiedliche Sensoreinrichtungen gibt, die in beliebiger Reihenfolge aufeinander folgen können.

Unter dem Begriff Messbereich sind die flächig oder räumlich begrenzten Bereiche, die die jeweilige Sensoreinrichtung erfassen kann, zu verstehen. Dabei kann ein solcher Messbereich über die gesamte Mattenbreite und/oder - dicke reichen.

Bevorzugt besitzt die erste Sensoreinrichtung einen Sensor, um den Metallanteil eines Fremdkörpers auszumachen. Mit einem solchen Sensor, beispielsweise einem durch elektromagnetische Spulen, also induktiv unterstützten Detektor, erfährt man sofort, wenn sich ein eisenhaltige Metallteil in der Matte befindet.

In Kombination mit einer ebenfalls bevorzugten zweiten Sensoreinrichtung, die geeignet ist, die Abmessungen des Fremdkörpermaterials vorzugsweise dreidimensional zu erfassen (hier eignen sich beispielsweise durch Röntgenstrahlung oder Radiometrie unterstützte Sensoren), kann die Gefährlichkeit des Fremdkörpers für das Pressenband bestimmt werden. So weiß der Betreiber der Produktionsanlage beispielsweise sofort, dass er die Produktion von Werkstoffplatten nicht unterbrechen muss, wenn der Metalldetektor zwar angeschlagen hat, die zweite Sensoreinrichtung aber das Signal gibt, dass es sich nur um verstreute winzige Metallpartikel handelt.

Die Ansprüche 4 bis 7 geben besonders sinnvolle Sensoreinrichtungen an. Diese Sensoreinrichtungen werden bereits in anderen technischen Bereichen, beispielsweise der Medizin oder der Lebensmitteltechnik angewendet und haben sich bewährt.

Es ist jedoch zudem besonders bevorzugt, dass der erste Sensor mit einer Ermittlungseinheit zur Messung einer Phasenverschiebung des durch einen detektierten Metallpartikel erzeugten Signals ausgerüstet oder verbunden ist, um auf diese Weise auch eine Identifizierung der Metallart zu ermöglichen, und so zwischen Eisenhaltigen und Nichteisenhaltigen Metallen unterscheiden zu können. Insbesondere ist so eine Identifizierung der Metallart möglich. Es kann zwischen Kupfer, Messing, Aluminium, Eisen und Stahl unterschieden werden, auf Grund der Dichten fallsweise sogar innerhalb der Arten in unterschiedliche Sorten bzw. Rubriken, beispielsweise zwischen Gusseisen oder Baustahl.

Mit besonderem Vorteil ist eine Auswerteeinrichtung vorgesehen, die in der Lage ist, eine Sensor-Fusion der zumindest einen ersten Sensoreinrichtung und der wenigsten einen zweiten Sensoreinrichtung vorzunehmen.

Die Sensorfusion kombiniert algorithmisch die Messergebnisse der zumindest einen ersten Sensoreinrichtung und der wenigsten einen zweiten Sensoreinrichtung und erstellt somit eine lückenlose sowie präzise Zustandsschätzung des Fremdkörpers. Sie kommt dann beispielsweise anhand einer vorgegebenen Schädlichkeitsmatrix zu dem Ergebnis, ob der Fremdkörper für das Pressenband schädlich ist oder nicht. Die Matrix gibt dazu beispielsweise in Abhängigkeit von Fremdkörpermaterial und Fremdkörperabmessungen eine klare Einstufung. Genauso können Abhängigkeitsalgorithmen hinterlegt sein mit Formeln, die einen kritischen Zustand der Fremdkörper bei Überschreiten eines errechneten Grenzwertes definieren.

Vorzugsweise ist einem Fall der eingeschätzten möglichen Schädlichkeit für das Pressenband, also in Abhängigkeit von dem Ergebnis der Sensor-Fusion in der Auswerteeinrichtung eine Fremdkörperausschleuseeinrichtung aktivierbar.

Eine Öffnung eines Ausschleusefallschachts oder die Aktivierung einer Absaugeinrichtung durch diese erfindungsgemäße Vorrichtung zur Detektion von Fremdkörpern geschieht demzufolge völlig automatisch. Der Betreiber der Produktionsanlage kann sich zudem sicher sein, dass die Produktionsunterbrechung nur bei potenziell das Pressenband schädigender Fremdkörpererkennung oder zumindest höchster Wahrscheinlichkeit einer solchen Schädigung stattfindet. So wäre es gemäß Auswerteeinrichtung beispielsweise in dem Fall, bei dem ein zwar großer, aber sehr flacher Fremdkörper in der Matte verborgen ist, welcher bei einem herkömmlichen Fremdkörperscanner bereits einen Produktionsstopp hervorrufen würde, möglich, die Produktionsanlage weiterlaufen zu lassen und gegebenenfalls den Bereich mit dem Fremdkörper aus der später entstehenden Werkstoffplatte herauszuschneiden. Für erkannte, aber für das Pressenband unschädliche Fremdkörper muss die Produktionsanlage demzufolge nicht angehalten werden.

Die Tendenz bei Werkstoffplatten geht dahin, immer dünnere und leichtere herstellen zu können. Diese sind von dem Problem besonders betroffen und die erfindungsgemäße Vorrichtung eignet sich besonders bei Mattendicken < 8 mm. Deshalb ist es von Vorteil, wenn zusätzlich zu der zumindest einen ersten Sensoreinrichtung und der wenigsten einen zweiten Sensoreinrichtung ein Dicken-, Dichte- oder Flächengewichtsmessgerät für die Matte vorhanden ist.

Derartige Dicken-, Dichte- oder Flächengewichtsmessgeräte sind in der Produktion von gestreuten Matten, die zu Werkstoffplatten gepresst werden, geläufig und dem Fachmann bekannt. In der Regel misst man damit aber nur die Gleichmäßigkeit über die Streuungshöhe und/oder über die Breite und längs des Förderbandes.

Bei der erfindungsgemäßen Vorrichtung kann der ermittelte Dicken- oder Dichte- oder Flächengewichtsmess jedoch ebenfalls an die Auswerteeinheit übermittelt werden. In der dort vorhandenen Auswertematrix zum Fremdkörpermaterial und zur Fremdkörperabmessung kann dieser Messwert mitberücksichtigt werden. Bei dünnen Matten können nämlich auch bereits kleinere Fremdkörper das Pressenband gefährden.

Mit besonderem Vorzug ist vorgesehen, dass zur Synchronisation der Sensorsignale der zumindest einen ersten Sensoreinrichtung und der wenigstens einen zweiten Sensoreinrichtung eine Weg-/Zeit-Erfassungseinrichtung vorgesehen ist.

Insbesondere wenn die zumindest eine erste Sensoreinrichtung und die wenigstens eine zweite Sensoreinrichtung längs der Transportstrecke der Matte ein Stück voneinander entfernt angeordnet sind, muss gewährleistet werden, dass die Messwerte der ersten Sensoreinrichtung und die Messwerte der zweiten Sensoreinrichtung von der gleichen Messstelle einander zugeordnet werden. Deshalb kommen die Messwerte der in Transportrichtung nachgeordneten Messstelle in Abhängigkeit von der Transportgeschwindigkeit der Matte erst später bei der Auswerteeinrichtung an. Dieser zeitliche Versatz muss berücksichtigt werden. Mit der Weg-/Zeit-Erfassungseinrichtung ist es möglich, diese Zeitdifferenz zu erfassen und an die Auswerteeinheit weiterzugeben. Vielfach ist der Abstand der Sensoreinrichtung festgelegt, so dass die Weg-/Zeit-Erfassungseinrichtung lediglich die Geschwindigkeit der Fördereinrichtung, also beispielsweise des Förderbandes, übermitteln. Dann kann die Auswerteeinrichtung den zeitlichen Versatz der Messsignale errechnen und bei der Analyse der Fremdkörper berücksichtigen.

Alternativ kann auch vorgesehen sein, dass die zumindest eine erste Sensoreinrichtung und die wenigstens eine zweite Sensoreinrichtung gleichzeitig die gleiche Messstelle auf bzw. in der Matte erfassen. In diesem Fall könnten sie sogar in einem Gehäuse untergebracht werden.

Weiterhin kann es mit besonderem Vorteil vorgesehen sein, dass die Auswerteeinrichtung zu einer Schadenshergangsanalyse ausgebildet ist.

Dadurch können sich große Synergieeffekte ergeben. Da die Vorrichtung wenigstens eine erste Sensoreinrichtung aufweist, die auf einen oder mehrere räumliche Messbereiche der Matte gerichtet ist, um Fremdkörper zu erkennen und wenigstens eine zweite Sensoreinrichtung aufweist, die ebenfalls auf die Messbereiche ausgerichtet ist, und wobei die erste Sensoreinrichtung geeignet ist, eine Materialerkennung des Fremdkörpers vorzunehmen, und die zweite Sensoreinrichtung geeignet ist, wenigstens eine Fremdkörperabmessung zu bestimmen, ergibt sich bei einer Ausgestaltung der Auswerteeinrichtung dahingehend, dass eine Schadenshergangsanalyse durchführbar ist die Möglichkeit, dass insbesondere aus einer Sensor-Fusion gewinnbare und insbesondere gewonnene Daten als Indizien einer durchzuführenden Schadenhergangsanalyse bereitstellbar sind und insbesondere bereitgestellt werden. Insgesamt ergibt sich damit der Vorteil, dass ein guter, in der Praxis bereits in vielen Fällen gut brauchbarer Grad an Aussagefähigkeit der Schadensermittlung erreichbar ist. Werden Fremdkörper einer bestimmten Größe und eines bestimmten Materials erfasst kann dies bereits auf bestimmte Schäden in der Produktion beziehungsweise in den für die Produktion genutzten Einrichtungen und/oder Anlagen hinweisen. Dazu kann es besonders aufschlussreich sein, wenn neben der Größe auch die Formen der Fremdkörper, insbesondere dreidimensional, erfasst werden. Dabei kann es vorgesehen sein, dass die Sensorik der Auswerteeinrichtung die Form in ausreichender Auflösung zur Verfügung stellt, um eine Kantenbetrachtung der Form des zu betrachtenden Fremdkörpers zuzulassen, die innerhalb der Schadenshergangsanalyse weitere Rückschlüsse auf mögliche Schadensbilder, insbesondere innerhalb der für die Produktion genutzten Einrichtung(en) und/oder Anlage(n) zulässt. Ebenso können Muster in der räumlichen und/oder zeitlichen Verteilung der Erfassung innerhalb der Schadenshergangsanalyse verarbeitbar sein, um den Grad der Aussagefähigkeit weiter zu erhöhen. So kann die Schadenshergangsanalyse beispielsweise derart ausgebildet sein, um pulsierend auftretende Muster von Fremdkörpern zuzuordnen und diese beispielsweise auch nach unterschiedlicher Kantenform zu Gruppieren, selbst wenn die erfassten Fremdkörper aus dem gleichen Material bestehen und etwa eine gleiche Größe aufweisen, können sie also innerhalb der Schadenshergangsanalyseeinheit einer anderen (Unter-)Gruppe zuzuordnen sein, aus der auf eine andere Gruppe möglicher Schadensursachen geschlossen wird. Die Schadenshergangsanalyse(-einheit) ist also vorzugsweise auch zur Gruppen- und/oder Matrixbildung ausgelegt.

Ergänzend oder alternativ kann es weiterhin von großem Vorteil sein, wenn die Vorrichtung derart fortgebildet ist, dass die Auswerteeinrichtung zur Schadensvorhersage ausgebildet ist.

Mit analogen Fortbildungen kann auch ein Zusammenwirken mit einer Schadensvorhersageeinheit vorgesehen sein, die für sich genommen Schäden aus den gewonnenen Daten in einem gewissen Grad einer Aussagefähigkeit vorhersagen kann. Besondere Synergien ergeben sich aber auch hier, wenn die Auswerteeinrichtung sowohl mit einer Schadenshergangsanalyse(-einheit) und einer Schadensvorhersage(-einheit) ausgestattet ist oder mit ihnen in Wirkverbindung steht und die beiden Analysen beziehungsweise Analyseeinheiten auch untereinander vernetzt sind.

Dann nämlich kann die Auswerteeinrichtung aus den über die wenigstens zwei Sensoren gewonnenen Signale (Daten) sowohl ein Schadensbild erkennen und insbesondere mit einem besonders hohen Grad an Aussagefähigkeit einer speziellen Einrichtung (Station) einer Anlage zur Herstellung von Werkstoffplatten zuordnen und voraussagen, wann der Schaden in der Zukunft etwa ein bestimmtes Maß erreicht haben wird. Der Betreiber einer solchen Anlage kann dann beispielsweise ein Maß festlegen, ab wann die Anlage zur Wartung heruntergefahren wird, um einen deutlich kostspieligeren Crash zu vermeiden. Auch kann der Betreiber dann - rechtzeitig informiert- entscheiden, ob er innerhalb einer vorgesehenen Wartungsmaßnahme frühzeitig ein detektierten Verschleiß erzeugendes Bauteil austauschen läßt.

Große Vorteile ergeben sich, insbesondere auch im Zusammenhang mit einem oder beiden der vorgenannten Ausgestaltungsvarianten, wenn die Auswerteeinrichtung eine lernfähige Einheit umfasst oder mit einer lernfähigen Einheit in Wirkverbindung steht.

Auf diese Weise ist eine ständige Verbesserung der Detektions- und Analysekompetenz der Vorrichtung möglich und es sind immer bessere Grade an Aussagefähigkeiten erreichbar. Vor allem ist das Vorsehen einer lernfähigen Einheit im hier beschriebenen Zusammenhang aber deswegen von Vorteil, weil bei der Herstellung von Werkstoffplatten häufig Rezepturen geändert werden müssen. Dies hat im Wesentlichen zwei Gründe: einerseits stehen zur Erzeugung einer bestimmten Art und Sorte von Werkstoffplatten nicht immer alle dazu notwendigen Rohstoffe in gleichbleibender Qualität und zu gleichbleibendem Preis zur Verfügung, sodass der Betreiber einer Anlage zur Werkstoffplattenherstellung bereits aus wirtschaftlichen oder technischen Gründen die Rezeptur seiner Werkstoffplatte über die Zeit meist recht häufig anpassen muss, manchmal sogar täglich. Zum anderen ist die Anschaffung einer Anlage zur Werkstoffplattenerzeugung mit hohen Kosten im zwei oder sogar im niedrigen dreistelligen Millioneneurobereich verbunden. Daraus ergibt sich, dass die Anlage lange Jahre, meist über wenigstens zwei bis drei Jahrzehnte, oft sogar länger, betrieben werden muss. Da aber die Anforderungen des Marktes sich über einen solchen Zeitraum erheblich ändern können, muss die Anlage auch derart gestaltet sein, dass es dem Betreiber möglich ist innerhalb einer Art von Werkstoffplatten, zum Beispiel Spanplatten, auch andere Sorten, z.B. Dünnplatten, mittelstarke Platten, Starkplatten in ein- oder mehrschichtiger Ausführung herzustellen, oder sogar verschiedene Arten von Werkstoffplatten, z.B. Spanplatten, Hybridplatten wie Spanplatten mit MDF-Deckschichten oder Ähnliches, zu erzeugen.

Die lernfähige Einheit kann dann helfen, gewonnene Erkenntnisse aus dem Herstellungsprozess der einen Sorte einer Art auch auf eine andere Sorte dieser Art oder sogar auf den Herstellungsprozess einer anderen Art von Werkstoffplatten, insbesondere innerhalb einundderselben Anlage, zu übertragen. Denn die detektierbaren Fremdkörper und Fremdkörpermuster müssen nicht zwangsweise aus Anlage, beziehungsweise einer Einrichtung und/oder Station der Anlage stammen, sondern können auch aus dem Herstellungsprozess erzeugt sein, insbesondere wenn es sich um Verklumpungen handelt oder in einem Rohstoff Fremdkörperpartikel vorhanden waren. Dies kann insbesondere bei der Verarbeitung von Recyclingmaterial der Fall sein. Zudem weisen Einjahrespflanzen, insbesondere Grasartige Pflanzen, häufig hohe Silikatanteile auf. Diese können im Aufbereitungsprozess, etwa im Bereich einer Aufspleißung, Zerfaserung und/oder Kochung zwar häufig recht gut vom Nutzanteil (Faseranteil) getrennt werden, aber sind nicht immer zwangsweise auch vollständig aus dem Massestrom innerhalb des Herstellungsprozesses entfernbar. Auch hier kann es zu ungewollten oder sogar gefährlichen Anhäufungen in der Matte kommen.

Zur Fortbildung der vorgenannten Ausführungsform ist es von ganz besonderem Vorteil, wenn die lernfähige Einheit zur Identifizierung, wenigstens möglicher, Schadensbilder auf Grund von Mustern von detektierten Signalen der einzelnen Sensoren ausgebildet ist.

Dies erhöht noch einmal deutlich den Mehrwert einer solchen Vorrichtung, da es sich in Versuchen überraschender Weise herausgestellt hat, dass gerade bei der Verwendung einer so ausgestalteten Vorrichtung im Zusammenhang mit der Herstellung von Werkstoffplatten zu unvorhersehbaren Verkürzungen der Analysezeiten kommen kann. Es ist nämlich nicht nur wichtig einen hohen Grad an Aussagefähigkeit zu erzielen, sondern es kann auch von großer Bedeutung sein, rechtzeitig eine Orientierung zur Sachlage zu erhalten. So kann die lernfähige Einheit beispielsweise pulsierend auftretende Muster umlaufenden Prozessen oder Einheiten zuordnen, an Hand des Wiederholungsabstandes kann dann auf die Länge des Umlaufes geschlossen werden. Werden in einem Prozess beziehungsweise in einer zur Ausführung eines Verfahrens ausgebildeten Anlage, beispielsweise einer Anlage zur Herstellung von Werkstoffplatten, verschiedene umlaufende Bänder (z.B. aus Stahl) verwendet, kann der Wiederholungsabstand zur Eingrenzung oder Zuordnung der geschädigten Einheit (Station) dienen.

Auch können unterschiedliche Rohmaterialien zur Bildung unterschiedlicher Schadensmuster neigen und bei entsprechender Ausbildung der lernfähigen Einheit als Hinweisgebende Indizien nutzbar sein.

Zudem ergeben sich große Vorteile, wenn die Vorrichtung mit wenigstens einem, einer Einrichtung/Station einer Produktionsanlage zur Herstellung von Werkstoffplatten zugeordneten Sensor in Wirkverbindung steht.

Auf diese Weise ist es möglich einen geschlossenes Informationsbild zu erhalten. Die Auswerteeinheit kann von den wenigstens zwei Sensoren detektierte Fremdkörper und insbesondere über einen bestimmten Zeitraum entstehende Muster von Fremdkörpern mit Signalen der verursachenden und/oder verarbeitenden Einheiten (Stationen) einer (Produktions-)Anlage zur Herstellung von Werkstoffplatten vergleichen. Besonders wertvoll ist es, wenn die Auswerteeinheit auch zur Sensor-Fusion mit wenigstens einem weiteren Sensor, insbesondere wenigstens einem, einer Einrichtung/Station einer Produktionsanlage zur Herstellung von Werkstoffplatten zugeordneten Sensor, ausgebildet ist.

Verfahrensmäßig sind die Vorteile analog zu den bezüglich der Vorrichtung Beschriebenen zu sehen. Die Verfahrensansprüche entsprechen im Wesentlichen den Vorrichtungsansprüchen zur Detektion von Fremdkörpern in einer auf eine Fördereinrichtung gestreuten Matte.

Entscheidend ist, dass sich auch im Verfahren deutliche Vorteile gegenüber dem Stand der Technik ergeben. Geht man beispielsweise von einem Metalldetektor als erster Sensoreinrichtung aus und von einer zweiten Sensoreinrichtung, die Abmessungen des Fremdkörpermaterials dreidimensional erfasst, so zeigen die drei folgenden Beispiele, wie effektiv eine Sensor-Fusion sein kann:
- Von der zweiten Sensoreinrichtung wird eine entsprechende Fläche oder Abmessung eines Körpers entdeckt. Mittels der ersten Sensoreinrichtung wird die Metall-Masse passend zur Körperfläche detektiert. Dann liegt sicher ein entsprechend gefährdender Metallkörper vor und eine Fremdkörperausschleusung wird aktiviert.
- Von der zweiten Sensoreinrichtung wird keine verdächtige Fläche oder Abmessung detektiert, der Metalldetektor meldet aber eine entsprechende Metall-Masse. Hier liegt dann ein größeres Feld von verteilten Metallstaub vor und kein gefährdender Fremdkörper. Eine Fremdkörperausschleusung kann unterbleiben.
- Von der zweiten Sensoreinrichtung wird eine entsprechende Fläche oder Abmessung eines Körpers entdeckt. Mittels der ersten Sensoreinrichtung wird kein Metall detektiert. Dann liegt sicher ein nicht-metallischer Körper vor (z.B. Leimklumpen) der über einer gesonderten Schwelle liegend entsprechend behandelt würde. Ab einer gewissen Abmessung, deren Wert auch von der Dicke der Matte und der Pressspalthöhe abhängig ist, wird eine Fremdkörperausschleusung aktiviert.

Die Ansprüche 22 und 23 sagen aus, dass es vorteilhaft ist, sowohl die erfindungsgemäße Vorrichtung als auch das analoge und entsprechende erfindungsgemäße Verfahren zur Detektion von Fremdkörpern in einer Produktionsanlage zwischen der Streustation und der kontinuierlichen Pressen einzusetzen.

Im Folgenden wird die Erfindung anhand von zwei Ausführungsbeispiele darstellenden Zeichnungen näher erläutert. Es zeigen
Fig. 1 eine Anlage zur Herstellung einer Werkstoffplatte im Sinne der Erfindung mit einer erfindungsgemäßen Vorrichtung zur Detektion von Fremdkörpern in einer schematischen Seitensicht,
Fig. 2 eine alternative Anordnung der Sensoreinrichtungen

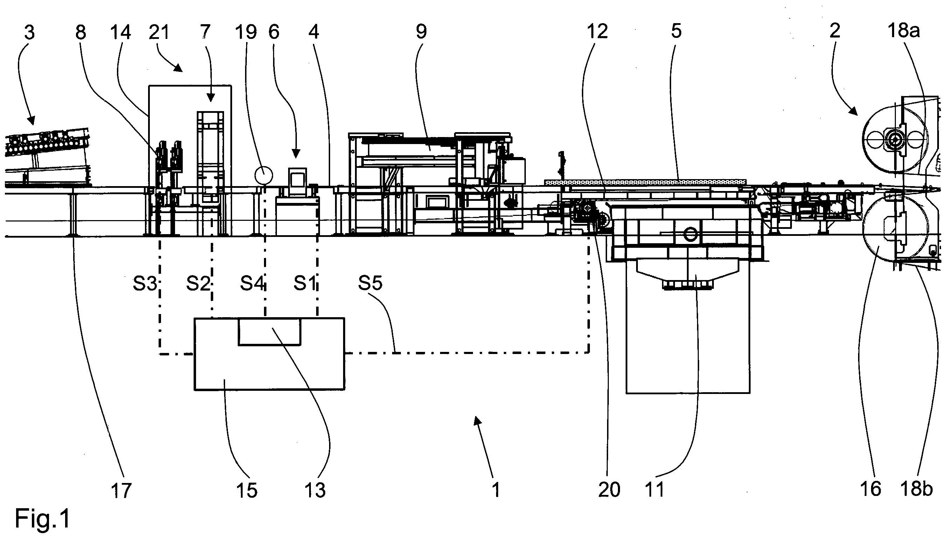

In Fig. 1 ist schematisch ein Ausschnitt einer Anlage 1 zur Herstellung einer Werkstoffplatte dargestellt.

Auf der linken Seite ist das Ende einer Streustation 3 angedeutet, mit der Späne oder Fasern, die mit Bindemitteln benetzt sind, auf eine lange Fördereinrichtung 4, in diesem Ausführungsbeispiel ein über eine Stützkonstruktion 17 laufendes Förderband, zu einer Matte 5 gestreut werden. Die Matte 5 kann mehrere unterschiedliche Lagen mit unterschiedlich großen Spänen bzw. Fasern haben, die ggf. auch noch in unterschiedliche Richtungen orientiert gestreut werden. In der Fig. 1 ist aus Gründen der Übersichtlichkeit nur ein kurzes Stück der Matte 5 schematisch dargestellt.

Am rechten Rand, also am Ende der Förderstrecke befindet sich der Einlauf in eine kontinuierliche Presse 2. Sie besitzt ein oberes Pressenband 18a und ein unteres Pressenband 18b, die um diverse Umlenkwalzen 16 endlos umlaufen und die Matte 5 von beiden Seiten mit Druck beaufschlagen können. Die Pressenbänder 18a, 18b sind dünne Metallbänder, die bei punktuellem Druck durch spitze oder harte Fremdkörper derart in Mitleidenschaft gezogen werden können, dass sich die Schäden auf die gepresste Matte 5 übertragen und beim (nicht dargestellten) Austritt aus der Presse 2 auf der Werkstoffplatte sichtbar sind.

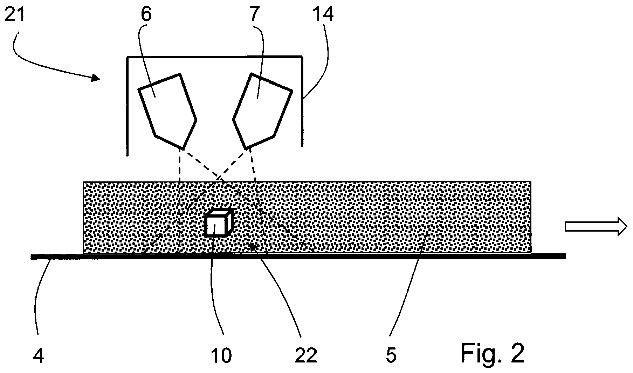

Das bedeutet, dass ein das Pressenband 18a, 18b gefährdende Fremdkörper 10 in der Matte 5 (in Fig. 2 näher dargestellt) unbedingt über eine Fremdkörperausschleuseeinrichtung 11 vor dem Einlauf in die kontinuierliche Presse 2 entfernt werden muss. In dem Ausführungsbeispiel ist die Fremdkörperausschleuseeinrichtung 11 eine Art Fallschacht, der durch ein Zurückziehen einer Abdeckung und/oder eines verfahrbaren Förderbandabschnitts 12 über Verstellmotoren 20 geöffnet werden kann. Nach dem Entfernen des Mattenabschnitts, der einen potenziell die Pressenbänder 18a, 18b gefährdenden Fremdkörper 10 enthält, ist keine gerade Kante der Matte 5 mehr vorhanden. Deshalb besitzt die Anlage 1 zur Herstellung einer Werkstoffplatte eine Diagonalsäge 9, die eine neue rechtwinklig zur Vorschubrichtung gerade neue Kante in die Matte 5 sägen kann. Diese Kante kann problemlos in die kontinuierliche Presse 2 einlaufen.

Das erfindungsgemäß Neue an dieser Anlage ist die Vorrichtung 21 und das Verfahren zur Detektion von Fremdkörpern in einer auf die Fördereinrichtung 4 gestreuten Matte 5. Entscheidend ist, dass eine erste Sensoreinrichtung 6 und eine zweite Sensoreinrichtung 7 zum Einsatz kommen. Die erste Sensoreinrichtung 6 ist geeignet, eine Materialerkennung des Fremdkörpers vorzunehmen, und die zweite Sensoreinrichtung 7 ist geeignet, die Fremdkörperabmessung zu bestimmen. Die Sensoreinrichtung 7 ist in diesem Ausführungsbeispiel der Sensoreinrichtung 6 vorgestaltet. In anderen Fällen arbeiten die beiden Sensoreinrichtungen 6, 7 in umgekehrter Reihenfolge, wie dies auch rein schematisch in Figur 2 angedeutet ist.

Aber zurück zu Fig. 1. Das physikalische Messsystem der Sensoreinrichtung 7 ist so ausgerüstet, dass es in der Lage ist, Abmessungen eines Fremdkörpers zu bestimmen, gegebenenfalls sogar dreidimensional. In diesem Ausführungsbeispiel kann es beispielsweise eine mit Röntgenstrahlen wirkende Sensoreinrichtung (7) sein. Im weiteren Vorlauf der Matte wird der gleiche räumliche Messbereich 22, in dem ein Fremdkörper 10 (siehe auch Fig. 2) größeren Ausmaßes festgestellt wurde, von der Sensoreinrichtung 6 untersucht. Diese Sensoreinrichtung 6 kann das Material des Fremdkörpers 10 oder zumindest dessen Dichte bestimmen, so dass aus den Abmessungen und den Materialkennwerten eine Aussage getroffen werden kann, ob der Fremdkörper 10 schädlich für die Pressenbänder 18a, 18b in der kontinuierlichen Presse 2 ist. Hier kann es schon genügen, wenn der Metallanteil des Fremdkörpers 10 bestimmt wird, was mit einem induktiv wirkenden Metalldetektor möglich ist.

Zur schnelleren Entscheidungsfindung wird eine Auswerteeinrichtung 15 vorgesehen, die über Signalleitungen S1 bis S5 verfügt. Über die Signalleitung S1 erhält sie Messwerte der ersten Sensoreinrichtung 6. Über die Signalleitung S2 erhält sie Messwerte der zweiten Sensoreinrichtung 7. Da die in der Auswerteeinrichtung 15 hinterlegten Fremdkörpergrenzwerte stark abhängig von der augenblicklich gefahrenen Mattendicke oder -dichte sind, werden entsprechende Daten über eine Signalleitung S3 von einem zusätzlich installierten Dicken-, Dichte- oder Flächengewichtsmessgerät 8 ergänzt. Um den zeitlichen Abstand eines Messbereichs 22 von der zweiten Sensoreinrichtung 7 bis zur ersten Sensoreinrichtung 6 bei den Messungen berücksichtigen zu können, wird die Auswerteeinrichtung 15 zudem von einem Förderbandgeschwindigkeitsmesser 19 mit Signalleitung S4 über eine Weg-/Zeit-Erfassungseinrichtung 13 informiert.

Stellt die Auswerteeinheit 15 einen die Pressenbänder 18a und 18b der kontinuierlichen Presse 2 bedrohenden Fremdkörper fest, so aktiviert sie Fremdkörperausschleuseeinrichtung 11.

In Fig. 2 sind die beiden Sensoreinrichtungen 6, 7 in umgekehrter Reihenfolge wir in Fig. 1 angeordnet. Allerdings sind die beiden Sensoreinrichtungen 6, 7 in einem Gehäuse 14 untergebracht und deshalb so dicht beieinander angeordnet, dass sich die Messbereiche 22 der Sensoreinrichtungen 6, 7 zumindest teilweise überlappen und beide ihre Messungen gleichzeitig ausführen können. In der Matte 5, die auf dem Förderband 4 transportiert wird, ist ein großer Fremdkörper 10 enthalten. Wenn man bei den gleichen Sensortypen wie im Ausführungsbeispiel der Fig. 1 bleibt, erkennt Sensoreinrichtung 6 das Material, beispielsweise Metall, und Sensoreinrichtung 7 identifiziert die Abmessungen, eventuell sogar dreidimensional, sodass die angeschlossene Auswerteeinrichtung anhand der gemessenen Werte und gespeicherter Grenzwerte sofort entscheiden kann, ob eine Fremdkörperausschleuseeinrichtung aktiviert werden muss. Allerdings soll die Erfindungen auch andere Messverfahren der Sensoreinrichtungen 6, 7 umfassen. Dazu gehören mit elektromagnetischen oder Ultraschall-Wellen oder mit Magnetfeldern oder mit Radiometrie arbeitende Sensoreinrichtungen.

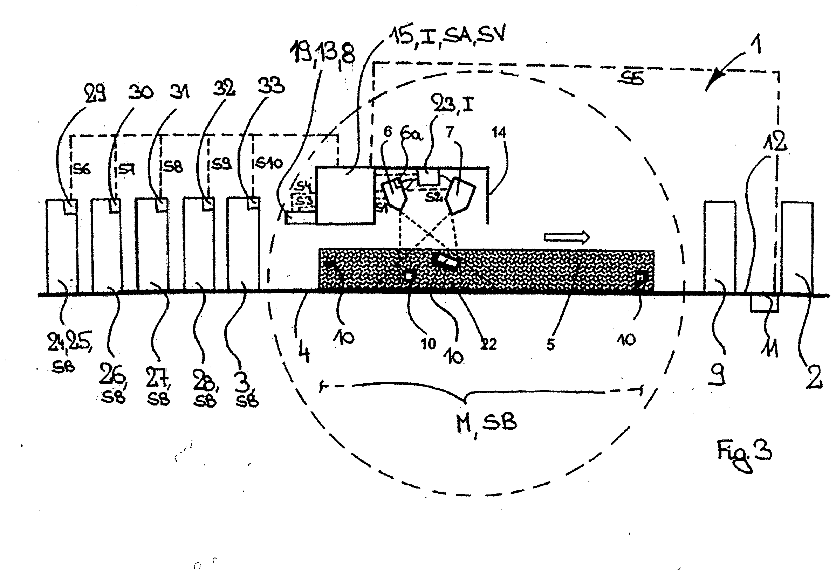

Figur 3 zeigt eine weitere Ausführungsform. Gleiche Bauteile und Elemente wie in Figur 1 und 2 tragen auch gleiche Bezugszeichen. Die in den Figuren gezeigten Ausführungsformen sind beispielhaft und im Rahmen der Offenbarung untereinander austauschbar. Figur 3 zeigt eine Ausführungsform einer Anlage 1 zur Herstellung einer Werkstoffplatte in einer größtenteils stark schematisierten Form. Die meisten Einrichtungen / Statione sind lediglich als kleine Kästchen dargestellt, wobei die Form und Größe der Kästchen nicht repräsentativ für die Zuordnung einer Relation des jeweiligen tatsächlichen Raumbedarfs der einzelnen Einrichtung/Station im Verhältnis zu einer anderen Einrichtung/Station entspricht. Im zentralen Bereich der Figur sind in einem kreisrunden, durch Strichlinien gebildeten Bereich, wichtige die Erfindung betreffende Merkmale in hervorgehobener, aber ebenfalls schematischen Darstellung, abgebildet. Dort ist erkennbar, dass die Auswerteeinrichtung 15 zu einer Schadenshergangsanalyse SA ausgebildet ist.

Dadurch können sich große Synergieeffekte ergeben. Da die Vorrichtung 21 wenigstens eine erste Sensoreinrichtung 6 aufweist, die auf einen oder mehrere räumliche Messbereiche 22 der Matte 5 gerichtet ist, um Fremdkörper 10 zu erkennen und wenigstens eine zweite Sensoreinrichtung 7 aufweist, die ebenfalls auf die Messbereiche 22 ausgerichtet ist, und wobei die erste Sensoreinrichtung 6 geeignet ist, eine Materialerkennung des Fremdkörpers 10 vorzunehmen, und die zweite Sensoreinrichtung 7 geeignet ist, wenigstens eine Fremdkörperabmessung zu bestimmen, ergibt sich bei einer Ausgestaltung der Auswerteeinrichtung 15 dahingehend, dass eine Schadenshergangsanalyse SA durchführbar ist die Möglichkeit, dass insbesondere aus einer Sensor-Fusion gewinnbare und insbesondere gewonnene Daten als Indizien einer durchzuführenden Schadenhergangsanalyse SA bereitstellbar sind und insbesondere bereitgestellt werden. Dazu kann es besonders aufschlussreich sein, wenn neben der Größe auch die Formen der Fremdkörper, insbesondere dreidimensional durch den zweiten Sensor 7 erfasst werden. Deswegen stellt die Sensorik der Auswerteeinrichtung 15 die erkannte Form in ausreichender Auflösung zur Verfügung, um eine Kantenbetrachtung der Form des zu betrachtenden Fremdkörpers zuzulassen, die innerhalb der Schadenshergangsanalyse SA weitere Rückschlüsse auf mögliche Schadensbilder SB, insbesondere innerhalb der für die Produktion genutzten Einrichtung(en) 24, 25, 26, 27, 28, 3, 4 und/oder Anlage(n) 1 zulässt. Zudem weist der erste Sensor 6 eine Ermittlungseinheit 6a zur Messung einer Phasenverschiebung des durch einen detektierten Metallpartikel erzeugten Signals auf oder ist mit ihr wie dargestellt verbunden, um auf diese Weise auch eine Identifizierung I der Metallart zu ermöglichen, und so zwischen Eisenhaltigen und Nichteisenhaltigen Metallen unterscheiden zu können, dabei kann der Sensor 6, mit Ermittlungseinheit 6a zwischen Kupfer, Messing, Aluminium, Eisen und Stahl unterschieden, auf Grund der Dichten sogar innerhalb der Arten in unterschiedliche Sorten bzw. Rubriken, beispielsweise zwischen Gusseisen oder Baustahl.

Ebenso können Muster M in der räumlichen und/oder zeitlichen Verteilung der Erfassung innerhalb der Schadenshergangsanalyse SA der Auswerteeinrichtung 15 verarbeitet werden, um den Grad der Aussagefähigkeit zu erhöhen. Die Schadenshergangsanalyse SA ist beispielsweise derart ausgebildet, um pulsierend auftretende Muster M von Fremdkörpern 10 zuzuordnen und diese beispielsweise auch nach unterschiedlicher Kantenform zu Gruppieren, selbst wenn die erfassten Fremdkörper 10 aus dem gleichen Material bestehen und etwa eine gleiche Größe aufweisen, können sie also innerhalb der Schadenshergangsanalyseeinheit SA einer anderen (Unter-)Gruppe zuzuordnen sein, aus der auf eine andere Gruppe möglicher Schadensursachen geschlossen wird. Die Schadenshergangsanalyse(-einheit) SA ist im dargestellten Beispiel also auch zur Gruppen- und/oder Matrixbildung ausgelegt. Zudem ist in der dargestellten Ausführungsform die Auswerteeinrichtung 15 auch (wenigstens wahlweise) zur Schadensvorhersage SV ausgebildet, wobei hier die beiden Analysen SA, SV beziehungsweise Analyseeinheiten SA, SV auch untereinander vernetzt sind. Auf diese Weise ist es besonders gut möglich, dass die Auswerteeinrichtung 15 aus den über die wenigstens zwei Sensoren 6, 7 gewonnenen Signale (Daten) S1, S2 sowohl ein Schadensbild SB erkennen und insbesondere mit einem besonders hohen Grad an Aussagefähigkeit einer speziellen Einrichtung (Station) 24, 25, 26, 27, 28, 3, 4 einer Anlage 1 zur Herstellung von Werkstoffplatten zuordnen und voraussagen, wann der Schaden in der Zukunft etwa ein bestimmtes Maß erreicht haben wird. Der Betreiber einer solchen Anlage 1 kann dann beispielsweise ein Maß festlegen, ab wann die Anlage 1 zur Wartung heruntergefahren wird, um einen deutlich kostspieligeren Crash zu vermeiden. Auch kann der Betreiber dann - rechtzeitig informiert- entscheiden, ob er innerhalb einer vorgesehenen Wartungsmaßnahme frühzeitig ein, einen detektierten Verschleiß erzeugendes, Bauteil austauschen läßt.

Die Auswerteeinrichtung 15 ist zudem mit einer lernfähigen Einheit 23 ausgestattet, und steht wie dargestellt mit dieser in Wirkverbindung.

Die lernfähige Einheit 23 kann sogar gewonnene Erkenntnisse aus dem Herstellungsprozess einer ersten Sorte einer Art von zu erzeugenden Werkstoffplatten auch auf eine andere Sorte dieser Art von zu erzeugenden Werkstoffplatten oder sogar auf den Herstellungsprozess einer anderen Art von Werkstoffplatten, insbesondere innerhalb ein und derselben Anlage übertragen. Denn die detektierbaren Fremdkörper 10 und (Fremdkörper-)Muster M müssen nicht zwangsweise aus der Anlage 1, beziehungsweise einer Einrichtung und/oder Station 24, 25, 26, 27, 28, 3, 4 der Anlage 1 stammen, sondern können auch aus dem Herstellungsprozess erzeugt sein, insbesondere wenn es sich um Verklumpungen handelt oder in einem Rohstoff Fremdkörperpartikel vorhanden waren. Um damit umgehen zu können ist die lernfähige Einheit 23 zur Identifizierung I, wenigstens möglicher, Schadensbilder SB auf Grund von Mustern M von detektierten Signalen S1 bis S10 der einzelnen Sensoren 6, 7, 8, 19, 29, 30, 31, 32, 33 ausgebildet ist. So kann die lernfähige Einheit 23 beispielsweise pulsierend auftretende Muster M umlaufenden Prozessen oder Einheiten zuordnen, an Hand des Wiederholungsabstandes kann dann auf die Länge des Umlaufes geschlossen werden. Werden in einem Prozess beziehungsweise in einer zur Ausführung eines Verfahrens ausgebildeten Anlage, verschiedene umlaufende Bänder (z.B. aus Stahl) verwendet, kann der Wiederholungsabstand zur Eingrenzung oder Zuordnung der geschädigten Einheit (Station) dienen. Auch können unterschiedliche Rohmaterialien zur Bildung unterschiedlicher Schadensmuster neigen und bei entsprechender Ausbildung der lernfähigen Einheit 23 in ihrer gezeigten Form als Hinweisgebende Indizien nutzbar sein. Zudem runden die aus den Sensor 29 bis 33 gewonnenen Signale S6 bis S10 die Effektivität der Auswerteeinrichtung 15 ab, da auf diese Weise auch bei Durchführung des beschriebenen Verfahrens ein geschlossenes Informationsbild erhalten werden kann. Die Auswerteeinheit 15 kann von den wenigstens zwei Sensoren 6, 7 detektierte Fremdkörper 10 und insbesondere über einen bestimmten Zeitraum entstehende Muster M von Fremdkörpern 10 mit Signalen der verursachenden und/oder verarbeitenden Einheiten (Stationen) einer (Produktions-)Anlage zur Herstellung von Werkstoffplatten vergleichen. Erkennbar sind zwei etwa quadratische Fremdkörper 10, die in Figur 3 zu einem pulsierenden Muster M gleicher Partikel gehören und hier aus einem Metall bestehen sollen und somit vor Erreichen der Presse 2 auszuschleusen sind. Gleiches gilt für den dazwischen gelagerten, etwas länglich geformten und nach schräg unten weisenden Fremdkörper 10, obwohl dieser nicht aus Metall gebildet ist, sondern ein Stück einer Dichtlippe aus einem Hartgummi oder einem Kunststoff darstellt.

Wegen seiner Größe und/oder wegen seiner Lage (relativ weit oben in der Matte 5, kann aber auch dieser Fremdkörper die Presse 2, insbesondere deren Bänder 18a und 18b gefährden. Um den zeitlichen Abstand zwischen den detektierten Fremdkörpern 10 berücksichtigen zu können, wird die Auswerteeinrichtung 15 zudem von einem Förderbandgeschwindigkeitsmesser 19 mit Signalleitung S4 über eine Weg-/Zeit-Erfassungseinrichtung 13 informiert. Der am linken Rand des vergrößerten Bereichs der Matte folgende, hier dunkel geformte Fremdkörper 10 ist durch ein Textilteil gebildet und ungefährlich. Die Auswerteeinrichtung 15 wird keinen Befehl zur Ausschleusung des betreffenden Mattenanteils geben.

**Bezugszeichenliste**

| | |
|---|---|
| 1 | Anlage zur Herstellung einer Werkstoffplatte |
| 2 | (Einlauf in) kontinuierliche Presse (Einrichtung/Station) |
| 3 | (Ende der) Streustation (Einrichtung/Station) |
| 4 | Fördereinrichtung, Förderband (Einrichtung/Station) |
| 5 | Matte (nur Ausschnitt) |
| 6 | Erste Sensoreinrichtung |
| 6a | Ermittlungseinheit (zur Phasenverschiebung) |
| 7 | Zweite Sensoreinrichtung |
| 8 | Dicken-, Dichte- oder Flächengewichtsmessgerät |
| 9 | Diagonalsäge |
| 10 | Fremdkörper |
| 11 | Fremdkörperausschleuseeinrichtung |
| 12 | Verfahrbare Abdeckung bzw. Förderbandabschnitt |
| 13 | Weg-/Zeit-Erfassungseinrichtung |
| 14 | Gehäuse der Sensoreinrichtungen |
| 15 | Auswerteeinrichtung |
| 16 | Umlenkwalze |
| 17 | Stützkonstruktion der Fördereinrichtung |
| 18a | Oberes Pressenband |
| 18b | Unteres Pressenband |
| 19 | Bandgeschwindigkeitsmesser |
| 20 | Verstellmotor |
| 21 | Vorrichtung zur Detektion von Fremdkörpern |
| 22 | Messbereich |
| 23 | lernfähige Einheit |
| 24 | Zerkleinerung (Einrichtung/Station) |
| 25 | Zermahlung (Einrichtung/Station) |
| 26 | Kochung (Einrichtung/Station) |
| 27 | Trocknung (Einrichtung/Station) |
| 28 | Beleimung (Einrichtung/Station) |
| 29 | Sensor |
| 30 | Sensor |
| 31 | Sensor |
| 32 | Sensor |
| 33 | Sensor |
| S1 | Signal(-leitung) erste Sensoreinrichtung |
| S2 | Signal(-leitung) zweite Sensoreinrichtung |
| S3 | Signal(-leitung) Dickenmessgerät |
| S4 | Signal(-leitung) Förderbandgeschwindigkeit |
| S5 | Signal(-leitung) zur Fremdkörperausschleuseeinrichtung |
| S6 | Signal(-leitung) Zerkleinerung |
| S7 | Signal(-leitung) Zermahlung |
| S8 | Signal(-leitung) Kochung |
| S9 | Signal(-leitung)Trocknung |
| S10 | Signal(-leitung) Beleimung |
| I | Identifizierung |
| M | Muster |
| SA | Schadenshergangsanalyse |
| SB | Schadensbild |
| SV | Schadensvorhersage |
| | |

## Patentansprüche

1. Vorrichtung zur Detektion von Fremdkörpern (21) in einer auf eine Fördereinrichtung (4) gestreuten Matte (5) im Zuge einer Werkstoffplattenherstellung, wobei die Matte (5) zumindest teilweise aus Fasern oder Spänen und Bindemitteln besteht, welche unter Temperaturerhöhung verpresst werden,
wobei zumindest eine mit elektromagnetischen oder Ultraschall-Wellen oder mit Magnetfeldern oder mit Radiometrie arbeitende erste Sensoreinrichtung (6) vorgesehen ist, die auf einen oder mehrere räumliche Messbereiche (22) der Matte gerichtet ist, um Fremdkörper (10) zu erkennen,
**dadurch gekennzeichnet, dass**
wenigstens eine zweite Sensoreinrichtung (7) vorgesehen ist, die ebenfalls auf die Messbereiche (22) ausgerichtet ist, und wobei die erste Sensoreinrichtung (6) geeignet ist, eine Materialerkennung des Fremdkörpers (10) vorzunehmen, und die zweite Sensoreinrichtung (7) geeignet ist, wenigstens eine Fremdkörperabmessung zu bestimmen.

2. Vorrichtung gemäß Anspruch 1, **dadurch gekennzeichnet, dass** die zumindest eine erste Sensoreinrichtung (6) geeignet ist, den Metallanteil des Fremdkörpermaterials zu erfassen.

3. Vorrichtung gemäß Anspruch 1, **dadurch gekennzeichnet, dass** die wenigstens eine zweite Sensoreinrichtung (7) geeignet ist, die Abmessungen des Fremdkörpermaterials dreidimensional zu erfassen.

4. Vorrichtung gemäß einem der Ansprüche 1 bis 3, **dadurch gekennzeichnet, dass** die zumindest eine erste Sensoreinrichtung (6) einen vorzugsweise induktiv wirkenden Metalldetektor umfasst.

5. Vorrichtung gemäß einem der Ansprüche 1 bis 4, **dadurch gekennzeichnet, dass** die wenigstens eine zweite Sensoreinrichtung (7) einen auf Röntgenstrahlen basierenden Sensor umfasst.

6. Vorrichtung gemäß einem der Ansprüche 1 bis 5, **dadurch gekennzeichnet, dass** die zumindest eine erste Sensoreinrichtung (6) oder die wenigstens eine zweite Sensoreinrichtung (7) einen auf einer Thermografie basierenden Sensor umfasst.

7. Vorrichtung gemäß einem der Ansprüche 1 bis 6, **dadurch gekennzeichnet, dass** die zumindest eine erste Sensoreinrichtung (6) oder die wenigstens eine zweite Sensoreinrichtung (7) einen auf Ultraschallwellen basierenden Sensor mit Schallreflektionsempfänger umfasst.

8. Vorrichtung gemäß einem der Ansprüche 1 bis 7, **dadurch gekennzeichnet, dass** eine Auswerteeinrichtung (15) vorgesehen ist, die in der Lage ist, eine Sensor-Fusion der zumindest einen ersten Sensoreinrichtung (6) und der wenigsten einen zweiten Sensoreinrichtung (7) vorzunehmen.

9. Vorrichtung gemäß Anspruch 8, **dadurch gekennzeichnet, dass** in Abhängigkeit von dem Ergebnis der Sensor-Fusion in der Auswerteeinrichtung (15) eine Fremdkörperausschleuseeinrichtung (11) aktivierbar ist.

10. Vorrichtung gemäß einem der Ansprüche 1 bis 9, **dadurch gekennzeichnet, dass** zusätzlich zu der zumindest einen ersten Sensoreinrichtung (6) und der wenigsten einen zweiten Sensoreinrichtung (7) ein Dicken-, Dichte- oder Flächengewichtsmessgerät (8) für die Matte (5) vorhanden ist.

11. Vorrichtung gemäß einem der Ansprüche 1 bis 10, **dadurch gekennzeichnet, dass** zur Synchronisation der Sensorsignale der zumindest einen ersten Sensoreinrichtung (6) und der wenigstens einen zweiten Sensoreinrichtung (7) eine Weg-/Zeit-Erfassungseinrichtung (13) vorgesehen ist.

12. Vorrichtung gemäß einem der Ansprüche 1 bis 10, **dadurch gekennzeichnet, dass** die zumindest eine erste Sensoreinrichtung (6) und die wenigstens eine zweite Sensoreinrichtung (7) in einem Gehäuse (14) untergebracht sind.

13. Vorrichtung gemäß einem der Ansprüche 8 bis 12, **dadurch gekennzeichnet, dass** die Auswerteeinrichtung (15) zu einer Schadenshergangsanalyse (SA) ausgebildet ist.

14. Vorrichtung gemäß einem der Ansprüche 8 bis 13, **dadurch gekennzeichnet, dass** die Auswerteeinrichtung (15) zur Schadensvorhersage (SV) ausgebildet ist.

15. Vorrichtung gemäß einem der Ansprüche 8 bis 14, **dadurch gekennzeichnet, dass** die Auswerteeinrichtung (15) eine lernfähige Einheit (23) umfasst oder mit einer lernfähigen Einheit (23) in Wirkverbindung steht.

16. Vorrichtung gemäß Anspruch 15, **dadurch gekennzeichnet, dass** die lernfähige Einheit (23) zur Identifizierung (I), wenigstens möglicher, Schadensbilder (SB) auf Grund von Mustern (M) von detektierten Signalen der einzelnen Sensoren (S1 bis S5) ausgebildet ist.

17. Vorrichtung gemäß einem der Ansprüche 8 bis 16, **dadurch gekennzeichnet, dass** die Vorrichtung mit wenigstens einem, einer Einrichtung/Station (2, 3, 4, 24, 25, 26, 27, 28) einer Produktionsanlage zur Herstellung von Werkstoffplatten zugeordneten Sensor (29, 30, 31, 32, 33) in Wirkverbindung steht.

18. Verfahren zur Detektion von Fremdkörpern (10) in einer auf eine Fördereinrichtung (4) gestreuten Matte (5) im Zuge einer Werkstoffplattenherstellung, wobei die Matte (5) zumindest teilweise aus Fasern oder Spänen und Bindemitteln besteht, welche unter Temperaturerhöhung in einer kontinuierlichen Presse (2) verpresst werden,
wobei zumindest eine mit elektromagnetischen oder Ultraschall-Wellen oder mit Magnetfeldern oder mit Radiometrie arbeitende erste Sensoreinrichtung (6) verwendet wird, die auf einen oder mehrere räumliche Messbereiche (22) der Matte (5) gerichtet ist, um Fremdkörper (10) zu erkennen,
**dadurch gekennzeichnet, dass**
wenigstens eine zweite Sensoreinrichtung (7) verwendet wird, die ebenfalls, gegebenenfalls zeitversetzt, auf die Messbereiche (22) ausgerichtet ist, wobei mit der ersten Sensoreinrichtung (6) eine Materialerkennung des Fremdkörpers (10) vorgenommen und mit der zweiten Sensoreinrichtung (7) wenigstens eine Fremdkörperabmessung bestimmt wird.

19. Verfahren gemäß Anspruch 18, **dadurch gekennzeichnet, dass** die zumindest eine erste Sensoreinrichtung (6) den Metallanteil des Fremdkörpermaterials erfasst.

20. Verfahren gemäß Anspruch 18 oder 19, **dadurch gekennzeichnet, dass** die wenigstens eine zweite Sensoreinrichtung (7) die Abmessungen des Fremdkörpermaterials dreidimensional erfasst.

21. Verfahren gemäß einem der Ansprüche 18 bis 20, **dadurch gekennzeichnet, dass** über eine Auswerteeinrichtung (15) eine Sensor-Fusion der Signale der zumindest einen ersten Sensoreinrichtung (6) und der wenigsten einen zweiten Sensoreinrichtung (7) vorgenommen wird.

22. Verfahren gemäß Anspruch 21, **dadurch gekennzeichnet, dass** in Abhängigkeit vom Ergebnis der Sensor-Fusion in der Auswerteeinrichtung (15) entschieden wird, ob eine Fremdkörperausschleuseeinrichtung (11) aktiviert wird.

23. Verfahren gemäß Anspruch 21 oder 22, **dadurch gekennzeichnet, dass** die Auswerteeinheit (15) über schädliche Fremdkörper (10) in Abhängigkeit von der Mattendicke oder Mattendichte alarmiert oder die Fremdkörperausschleuseeinrichtung (11) aktiviert.

24. Verfahren gemäß einem der Ansprüche 18 bis 23, **dadurch gekennzeichnet, dass** die zumindest eine erste Sensoreinrichtung (6) und die wenigstens eine zweite Sensoreinrichtung (7) unterschiedliche physikalische Arbeitsmethoden nutzen.

25. Verfahren gemäß einem der Ansprüche 18 bis 24, **dadurch gekennzeichnet, dass** eine Weg-/Zeit-Erfassungseinrichtung (13) genutzt wird, um die Signale für den gleichen Messbereich (22) zu synchronisieren.

26. Verfahren gemäß einem der Ansprüche 18 bis 25, **dadurch gekennzeichnet, dass** die zumindest eine erste Sensoreinrichtung (6) und die wenigstens eine zweite Sensoreinrichtung (7) gleichzeitig denselben Messbereich (22) erfassen.

27. Verfahren gemäß einem der Ansprüche 21 bis 26, **dadurch gekennzeichnet, dass** unter Verwendung der Auswerteeinrichtung (15) eine Schadenshergangsanalyse (SA) durchgeführt wird.

28. Verfahren gemäß einem der Ansprüche 21 bis 27, **dadurch gekennzeichnet, dass** unter Verwendung der Auswerteeinrichtung (15) eine Schadensvorhersage (SV) durchgeführt ist.

29. Verfahren gemäß einem der Ansprüche 21 bis 28, **dadurch gekennzeichnet, dass** eine, der Auswerteeinrichtung (15) zugehörige oder mit dieser in Wirkverbindung stehende, lernfähige Einheit (23) mit Signalen (S1-S10) der Sensoren (6, 7, 29, 30, 31, 32, 33) versorgt und insbesondere geschult wird.

30. Verfahren gemäß Anspruch 29, **dadurch gekennzeichnet, dass** die lernfähige Einheit (23) zur Identifizierung (I), wenigstens möglicher, Schadensbilder (SB) auf Grund von Mustern (M) von detektierten Signalen der einzelnen Sensoren (S1 bis S5) genutzt wird.

31. Verfahren gemäß einem der Ansprüche 18 bis 30, **dadurch gekennzeichnet, dass** das Verfahren unter wenigstens temporärer Verwendung von Signalen (S6, S7, S8, S9, S10) von wenigstens einem, einer Einrichtung/Station (2, 3, 4, 24, 25, 26, 27, 28) einer Produktionsanlage zur Herstellung von Werkstoffplatten, zugeordneten Sensor (29, 30, 31, 32, 33) ausgeführt wird.

32. Produktionsanlage zur Herstellung von Werkstoffplatten mit einer Streustation (3) zur Streuung einer Matte (5) auf eine Fördervorrichtung (4), wobei die Matte (5) zumindest teilweise aus Fasern oder Spänen und Bindemitteln besteht, und einer kontinuierlichen Presse (2) zur Verdichtung der Matte (5),
**dadurch gekennzeichnet, dass** zwischen der Streustation (3) und der kontinuierlichen Presse (2) eine Vorrichtung zur Detektion von Fremdkörpern (21) gemäß einem der Ansprüche 1 bis 17 vorhanden ist.

33. Herstellungsverfahren für Werkstoffplatten mit einer Streustation (3), in der eine Matte (5) auf eine Fördervorrichtung (4) gestreut wird, wobei die Matte (5) zumindest teilweise aus Fasern oder Spänen und Bindemitteln besteht und in einer kontinuierlichen Presse (2) verdichtet wird,
**dadurch gekennzeichnet, dass** zwischen der Streustation (3) und der kontinuierlichen Presse (2) ein Verfahren zur Detektion von Fremdkörpern gemäß einem der Ansprüche 18 bis 31 angewendet wird.
